# EUROPÄISCHE PATENTSCHRIFT

(11) **EP 3 192 515 B1**
(45) Veröffentlichungstag und Bekanntmachung des Hinweises auf die Patenterteilung: **21.11.2018**
(21) Anmeldenummer: 17150991.2
(22) Anmeldetag: 11.01.2017
(51) Int. Cl.: A61K 31/575, A61K 47/10, A61K 47/44

(54) **PHARMAZEUTISCHE ZUSAMMENSETZUNG ENTHALTEND 7BETA-HYDROXYCHOLESTEROL GEEIGNET ZUR INTRAVENÖSEN VERABREICHUNG**
PHARMACEUTICAL COMPOSITION COMPRISING 7BETA- HYDROXYCHOLESTEROL SUITABLE FOR INTRAVENOUS ADMINISTRATION
COMPOSITION PHARMACEUTIQUE CONTENANT 7BETA- HYDROXYCHOLESTEROL ADAPTÉE À L'ADMINISTRATION INTRAVENEUSE

(30) Priorität: 12.01.2016 EP 16000071
(43) Veröffentlichungstag der Anmeldung: 19.07.2017
(73) Patentinhaber: Immunopharm AG, 9496 Balzers (LI)
(72) Erfinder: BIEBERSCHULTE, Werner, 9492 Eschen (LI); GRIMM, Christine, 78262 Gailingen (DE); ROHAN, Charles Fernando, 69120 Heideldberg (DE)
(74) Vertreter: Schüssler, Andrea

(56) Entgegenhaltungen:
- EP-A1- 2 522 350
- LARSSON ET AL: "In vivo interconversion of 7beta-hydroxycholesterol and 7-ketocholesterol, potential surrogate markers for oxidative stress", FREE RADICAL BIOLOGY AND MEDICINE, ELSEVIER INC, US, Bd. 43, Nr. 5, 27. Juli 2007 (2007-07-27), Seiten 695-701, XP022173778, ISSN: 0891-5849, DOI: 10.1016/J.FREERADBIOMED.2007.04.033
- CAPRIO DE J ET AL: "BILE ACID AND STEROL SOLUBILIZATION IN 2-HYDROXYPROPYL-BETA- CYCLODEXTRIN", JOURNAL OF LIPID RESEARCH, AMERICAN SOCIETY FOR BIOCHEMISTRY AND MOLECULAR BIOLOGY, INC, US, Bd. 33, Nr. 3, 1. März 1992 (1992-03-01), Seiten 441-443, XP000573769, ISSN: 0022-2275

## Beschreibung

### Gebiet der Erfindung

Die vorliegende Erfindung betrifft eine pharmazeutische Zusammensetzung umfassend (a) 7β-Hydroxycholesterol, (b) mindestens ein wasserlösliches organisches Lösungsmittel und (c) einen Lösungsvermittler, wobei der Lösungsvermittler PEG hydriertes Rizinusöl oder polyoxyethylierte 12-Hydroxystearinsäure ist; und ein Verfahren zur Herstellung davon. Diese Zusammensetzung eignet sich zur Herstellung einer intravenös zu verabreichenden Zubereitung und umfasst die pharmazeutisch aktive Substanz 7β-Hydroxycholesterol, die in Wasser schlecht löslich ist.

### Stand der Technik

Zur Herstellung von pharmazeutischen Zusammensetzungen, um das pharmazeutisch aktive Mittel zu dispergieren, so dass die Zusammensetzung an einen Patienten verabreicht werden kann, wird ein geeignetes Lösungsmittel oder Trägersystem benötigt. Das Lösungsmittel muss in der Lage sein, eine therapeutisch wirksame Menge des Wirkstoffs zu lösen oder aufzulösen, um eine wirksame Zusammensetzung zu erzeugen. Jedoch sind viele pharmazeutisch wirksame Verbindungen nicht ausreichend löslich in Lösungsmitteln, wie z.B. Wasser. Ein weiteres Problem ist, dass zahlreiche pharmazeutische Wirkstoffe nach der Verdünnung für eine Infusionslösung instabil werden oder sie weisen eine Zersetzung oder einen Aktivitätsverlust des Wirkstoffes während der Lagerung in einem LösungsmittelSystem auf. Die geringe Löslichkeit und die Anfälligkeit für eine Zersetzung limitiert die Verwendung dieser schwer wasserlöslichen pharmazeutisch aktiven Substanzen in der Therapie erheblich.

Die meisten der derzeit verfügbaren Verabreichungsformen der pharmazeutisch aktiven Substanz 7β-Hydroxycholesterol sind Kapseln in oraler Form, da das 7β-Hydroxycholesterol nicht wasserlöslich ist.

Es gibt aber auch den Ansatz 7β-Hydroxycholesterol in wasserlöslicher Form herzustellen. Die DE 35 07 721 A1 beschreibt ein Verfahren zur Darstellung wasserlöslicher Derivate des 7β-Hydroxycholesterols. Um die Derivate wasserlöslich zu machen, wurde die Umsetzung derselben mit Bernsteinsäureanhydrid durchgeführt und aus dem entstandenen Hemisuccinat wurde entweder das Natrium- oder das Kalium-Salz gebildet.

Christ et al. (Anticancer Res. 1991, 11(1):359-64) beschreiben die Herstellung von Phosphodiestern von 7β-Hydroxycholesterol. Diese beiden Salze haben eine anti-Tumor-Aktivität und sind zwar wasserlöslich, aber haben den Nachteil in wässriger Lösung nicht stabil zu bleiben.

Die EP 0 07 834 A1 betrifft wasserlösliche Derivate von Cholesterin, insbesondere wasserlösliche Derivate von 7-Hydroxycholesterol. Dies wird durch Verbindungen und Komplexen von Albumin mit organischen zweibasigen Säure-Halbestern von 7-Hydroxycholesterol erreicht.

EP 2 522 350 A1 betrifft die Verwendung einer Wirkstoffkombination aus einem 1-Diethylaminoethyl-3-chinoxalin-2-on-Derivat und einem Oxysterol zur Durchbrechung von Resistenzen bei der Behandlung von Krebs und zur Erhöhung der Immunleistungsfähigkeit bei Krebs, bakteriellen und viralen Erkrankungen, Autoimmunerkrankungen, erhöhter Stress- und Umweltbelastung.

De Caprio et al., J. of Lipid Research, American Socielty for Biochemistry and Molecular Biology, Bd. 33, Nr.3 (1992), S. 441-443 beschreiben die Stabilität von Sterolen, insbesondere 7-alpha-Hydroxycholesterol, in Wasser als Lösungsmittel und 2-Hydroxypropyl-beta-cyclodextrin als Lösungsvermittler.

Larsson et al., Free Radical Biology & Medicine 43 (2007), pp. 695-701 beschreiben die invivo Umwandlung von 7β-Hydroxycholesterol und 7-ketocholesterol, welche zwei potentielle Surogatmarker für oxidativen Stress sind.

Ein Nachteil des Standes der Technik ist, dass bisher die körpereigene Signalsubstanz 7β-Hydroxycholesterol nicht in wässriger Lösung eingesetzt werden konnte, sondern nur zwei wasserlösliche Salze derselben (Hydroxycholesterolbishemisuccinat-diethanolamin und 7β-Hydroxycholesterolbishemisuccinat-dinatrium). So beschreiben Maier et al. (Anticancer Res. 1999, 19(5b), S. 4251-4256) die Induktion von Apoptose durch beiden genannten Salze in menschlichen Kolonkarzinomzelllinien. Diese beiden Salze sind zwar wasserlöslich, aber in wässriger Lösung nicht stabil. Nach einigen Stunden beginnt die Substanz sich zu zersetzen und auszuflocken. Des Weiteren ist nicht bekannt, wie sich das Bishemisuccinat im Gegensatz zum 7β-Hydroxycholesterol verhält und welche zusätzlichen Nebenwirkungen von Ethanolamin bei der Anwendung im Menschen ausgehen können.

Ein weiterer Nachteil des Standes der Technik ist, dass 7β-Hydroxycholesterol in oraler Form nicht gerne verwendet wird, da nicht bekannt ist, wie viel von der Substanz am Zielort ankommt. Ebenfalls ist die Resorption des 7β-Hydroxycholesterol im Magen-Darm-Trakt nicht bekannt.

### Aufgabe der Erfindung

Daher besteht für eine gezielte pharmazeutische Anwendung der Bedarf, die Substanz 7β-Hydroxycholesterol in einer wässrigen Flüssigkeit in Lösung zu bringen bzw. zu halten und dann intravenös, z.B. per Infusion, verabreichen zu können.

### Ausführliche Beschreibung der Erfindung

Die vorliegende Erfindung löst das technische Problem durch die Bereitstellung einer Zusammensetzung, umfassend die pharmazeutisch aktive Substanz 7β-Hydroxycholesterol und ein Lösungsmittelsystem, das ein Lösungsmittel und einen Lösungsvermittler umfasst, wobei der Lösungsvermittler PEG hydriertes Rizinusöl oder polyoxyethylierte 12-Hydroxystearinsäure ist.

Erfindungsgemäß wird die Aufgabe durch eine pharmazeutische Zusammensetzung enthaltend
(a) 7β-Hydroxycholesterol
(b) ein wasserlösliches organisches Lösungsmittel
(c) einen nicht-ionischen Lösungsvermittler, wobei der Lösungsvermittler PEG hydriertes Rizinusöl oder polyoxyethylierte 12-Hydroxystearinsäure ist,
gelöst.

In einer bevorzugten Ausführungsform wird die Aufgabe durch eine pharmazeutische Zusammensetzung enthaltend
(a) 7β-Hydroxycholesterol
(b) Propylenglykol und/oder DMSO
(c) PEG hydriertes Rizinusöl
gelöst.

7β-Hydroxycholesterol (C₂₇H₄₆O₂) ist eine universell vorkommende Signalsubstanz mit einem Molekulargewicht von 402.65294 g/mol, die in der Thymusdrüse produziert wird und eine anti-proliferatorische Wirkung hat sowie eine universelle Signalsubstanz der körpereigenen Immunabwehr ist. Es wird daher vornehmlich zur Krebstherapie verschiedenster Tumoren und zur allgemeinen Steigerung der Immunabwehr eingesetzt. Es ist ein Stoffwechselprodukt, das durch Cholesterinoxidation entsteht. Als Oxysterole werden im Allgemeinen die sauerstoffhaltigen Abkömmlinge des Cholesterins (Cholesterol) bezeichnet. Die Sauerstoff-Funktionen (Hydroxy-Gruppen, Keto-Gruppen oder Epoxy-Gruppen) sind dabei an den Kohlenstoffatomen des Cholesterol-Grundgerüsts lokalisiert. 7β-Hydroxycholesterol ist durch die Strukturformel 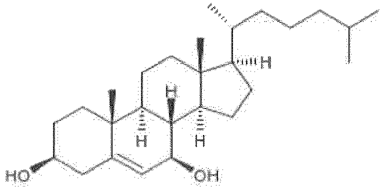 gekennzeichnet.

Ein geeignetes wasserlösliches organisches Lösungsmittel ist dem Fachmann hinreichend bekannt. Erfindungsgemäß bevorzugte wasserlösliche organische Lösungsmittel sind Polyethylenglykol 300, Polyethylenglykol 400, (Poly)propylenglykol, Aceton, Alkohole (z.B. Ethanol, n-Propanol, Isopropanol), Glyzerin, N-methyl-2-pyrrolidon, Dimethylacetamid oder Dimethylsulfoxid.

Bevorzugt ist die Verwendung von Propylenglykol (1,2-Propandiol; C₃H₈O₂) als wasserlösliches organisches Lösungsmittel. Propylenglykol ist als Lösungsmittel bekannt und wird erfindungsgemäß als solches für 7-Hydroxycholesterol verwendet. Es stellt eine klare, farblose, nahezu geruchlose und stark hygroskopische Flüssigkeit mit einem Molekulargewicht von 76,10 g/mol dar. Propylenglykol gehört zu den mehrwertigen Alkanolen. Es ist mit Wasser und Ethanol mischbar, mit fetten Ölen dagegen nicht. Bei hohen Temperaturen oberhalb 150 °C ist es oxidationsempfindlich. Propylenglykol ist eine chirale Verbindung (Stereozentrum an C2), die zumeist als Racemat eingesetzt wird. Vorteilhaft ist, dass Propylenglykol die Löslichkeit verschiedener Stoffe deutlich verbessert und eine stabilere Dispersion von Arzneistoffen gewährleisten kann. Darüber hinaus kann es häufig zu einer deutlichen Resorptionsverbesserung verschiedener Wirkstoffe beitragen. Die antimikrobielle Wirksamkeit macht einen Einsatz weiterer Konservierungsmittel häufig überflüssig. Desweiteren ist von Propylenglykol bekannt, dass es in Injektionsformulierungen vieler Arzneimittel enthalten ist, aber in bestimmten Konzentrationen lokale Unverträglichkeiten hervorrufen kann (siehe Kruss B. (Acta Pharm. Technol. 35(4) (1989) 187-196)). Es kann zu schmerzhaften Schwellungen und Gewebeunverträglichkeiten an der Injektionsstelle kommen, die sich zum Teil erst nach mehreren Wochen zurückbilden. Das Irritationspotential an der Haut ist in starkem Maße konzentrationsabhängig, ein geringer Zusatz wird aber allgemein als tolerierbar angesehen. Daher sollte der Einsatz in angemessener Konzentration erfolgen. Erfindungsgemäß wird in der zuzubereitenden Infusionslösung die die Irritationen hervorrufende kritische Konzentration (10-15 %) unterschritten und daher vom Patienten hervorragend vertragen. Propylenglykol ist durch die Strukturformel 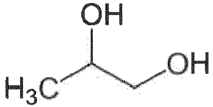 gekennzeichnet.

In einer bevorzugten Ausführungsform kann alternativ oder zusätzlich zu Propylenglykol Ethanol und/oder Aceton und/oder Dimethylsulfoxid (DMSO; C₂H₆OS) als Lösungsmittel verwendet werden. DMSO ist ein organisches Lösungsmittel und zählt zur Klasse der Sulfoxide. Es ist in jedem Verhältnis mit Wasser sowie vielen organischen Lösungsmitteln mischbar. DMSO selbst hat eine pharmazeutische Wirkung (antiphlogistisch und analgetisch) und wird oft in Salben, Gelen, Tinkturen oder Pflastern zur perkutanen Behandlung stumpfer Verletzungen und lokaler Schmerzzustände eingesetzt.

7β-Hydroxycholesterol weist in einem wasserlöslichen organischen Lösungsmittel (z.B. Propylenglykol und/oder DMSO und/oder Ethanol und/oder Aceton) eine hervorragende Löslichkeit auf und ermöglicht diese Lösung über Jahre stabil zu halten. Will man jedoch die 7β-Hydroxycholesterol/Lösungsmittel-Lösung in einer wässrigen Flüssigkeit, z.B. zur Bereitstellung einer Infusionslösung, verdünnen, flockt die Substanz aus. Von den Erfindern wird daher ein Lösungsvermittler eingesetzt, um das 7β-Hydroxycholesterol in der späteren Infusionslösung in Lösung zu halten.

Erfindungsgemäß ist der Lösungsvermittler ein nicht-ionischer Oberflächen-aktiver Stoff (Surfactant). Erfindungsgemäß bevorzugte Lösungsvermittler sind ausgewählt aus PEGhydriertem Rizinusöl (Cremophor®-Serie), D-Tocopherol Polyethylenglykol 1000-Succinat (Kolliphor® TPGS), Polysorbat 20 (Tween® 20), Polysorbat 80 (Tween® 80), polyoxyethylierter 12-Hydroxystearinsäure (Kolliphor® HS 15; Solutol® HS15), Sorbitanmonooleoat (Span® 20), Poloxamer 407 (Kolliphor® P 407), Poloxamer 188 (Kolliphor® P 188), PEG 300 Caprylsäure/Capringlyceride (Softigen® 767), PEG 400 Caprylsäure/Capringlyceride (Labrasol®), PEG 300 Ölsäureglyceride (Labrafil® M-1944CS), PEG 300 Linolsäureglyceride (Labrafil® M-2125CS), Lauroylpolyoxyglyceride (Gellusire® 44/14), n-Decansäureester mit 1,2,3-propantrioloctanoat (Softigen® 767), Mono- oder Difettsäureester von PEG 330, 400 oder 1750.

Als besonders geeigneter Lösungsvermittler hat sich erfindungsgemäß ein PEG hydriertes Rizinusöl herausgestellt. Es konnte insbesondere gezeigt werden, dass durch die Verwendung von PEG-40 hydriertem Rizinusöl eine stabilisierte flüssige pharmazeutische Formulierung hergestellt werden konnte.

PEG hydriertes Rizinusöl ist ein Polyethylenglykol (PEG)-Derivat von hydriertem Rizinusöl und ist eine bernsteinfarbene, leicht viskose Flüssigkeit. Rizinusöl ist ein Pflanzenöl, das aus den Samen des tropischen Wunderbaums (*Ricinus communis*), eines Wolfsmilchgewächses, gewonnen wird. Es besteht aus verschiedenen Triglyceriden und wird in der Pharmazie auch "Polyoxyethylated castor oil" genannt. Bei der Hydrierung des Rizinusöls wird an die Doppelbindung der Rizinolsäure Wasserstoff unter Verwendung von Raney-Nickel addiert. Die Hydroxygruppe bleibt erhalten und es entsteht ein Fett (Rizinuswachs), das Triglycerid der 12-Hydroxystearinsäure. Polyethylenglykole (PEG) sind ein synthetischer Rohstoff, dessen Basisbaustein das Ethylenoxid (C₂H₄O) ist. Mit Wasser entsteht aus Ethylenoxid das Ethylenglykol, das als Frostschutzmittel bekannt ist. Ethylenglykol kann mit Ethylenoxid weiterreagieren und dadurch Diethylenglykol und schließlich Polyethylenglykole (PEG) unterschiedlicher Länge mit entsprechend vielfältigen Eigenschaften bilden.

Handelsprodukte des PEG hydrierten Rizinusöls sind beispielsweise auf der Basis von PEG7, PEG25, PEG35, PEG40, PEG50 und PEG60 bekannt: Croduret™ 25 (Fa. Croda), Croduret™ 40 (Fa. Croda), Croduret™ 50 (Fa. Croda), Croduret™ 60 (Fa. Croda) Cremophor® RH 40 (Fa. BASF), Cremophor® RH 60 (Fa. BASF), Cremophor® RH 410 (Fa. BASF), Kolliphor EL bzw. Cremophor EL (Fa. BASF), Emulgin® HRE 40 (Fa. BASF), Emulgin® HRE 455 (Fa. BASF) und EMAROL H40 (Fa. CISME, Italien). Erfindungsgemäß eignet sich PEG40 hydriertes Rizinusöl (Cremophor® RH40) besonders.

Als weiterer besonders geeigneter Lösungsvermittler hat sich erfindungsgemäß eine polyoxyethylierte 12-Hydroxystearinsäure herausgestellt. Es konnte insbesondere gezeigt werden, dass durch die Verwendung von polyoxyethylierter 12-Hydroxystearinsäure eine stabilisierte flüssige pharmazeutische Formulierung hergestellt werden konnte.

Handelsprodukte der polyoxyethylierten 12-Hydroxystearinsäure sind Kolliphor® HS15 (Fa. BASF) bzw. Solutol® HS15. Kolliphor® HS15 besteht aus Polyglykol-Monoestern und - Diestern von 12-Hydroxystearinsäure (=lipophiler Teil) und ungefähr 30% freiem Polyethylenglykol (= hydrophiler Teil).

Erfindungsgemäß enthält die pharmazeutische Zusammensetzung
a) 7β-Hydroxycholesterol 0,1-2,0 Gew.-%, bevorzugt 0,3-1,5 Gew.-%, ganz bevorzugt 0,5-1,3 Gew.-%, insbesondere bevorzugt 02, 0,3, 0,4, 0,5, 0,6, 0,7, 0,8, 0,9, 1,0, 1,1, 1,2, 1,3, 1.4 oder 1,5 Gew.-%
b) Wasserlösliches organisches Lösungsmittel (z.B. Propylenglykol und/oder DMSO und/oder Ethanol und/oder Aceton) 68-97 Gew.-%, bevorzugt 70-95 Gew.-%, ganz bevorzugt 75-90 Gew.%, insbesondere bevorzugt 70, 71, 72, 73, 74, 75, 76, 77, 78, 80, 81, 82, 83, 84, 85, 86, 87, 88, 89, 90, 91, 92, 93, 94 oder 95 Gew.%
c) Lösungsvermittler (z.B. PEG hydriertes Rizinusöl oder polyoxyethylierte 12-Hydroxystearinsäure) 1-30 Gew.-%, bevorzugt 5-25 Gew.-%, ganz bevorzugt 5-20 Gew.-%, ganz besonders bevorzugt 5, 6, 7, 8, 9, 10, 11, 12, 13, 14, 15, 16, 17, 18, 19 oder 20 Gew-%.

Die Zusammensetzung kann weiter übliche pharmazeutisch verträgliche Zusätze enthalten, z.B. Alkohole (vorzugsweise 1-15 Gew-% Ethanol) oder Antioxidantien (vorzugsweise 1-10 Gew-% Caroverinhydrochlorid, 1-10 Gew-% Glutathion, 1-10 Gew.-% Vitamin C). Bevorzugt kann die Zusammensetzung desweiteren wasserunlösliche Lipide (z.B. Rizinusöl, Maiskeimöl, Baumwollsamenöl, Olivenöl, Erdnussöl, Pfefferminzöl, Safloröl, Sesamöl, Sojabohnenöl, hydrogenierte Pflanzenöle, mittelkettige Triglyceride von Kokosnussöl und Palmöl), organische Flüssigkeiten/Halbfeste Stoffe (z.B. Bienenwachs, D-Tocopherol, Ölsäure, mittelkettige Mono- oder Diglyceride), Cyclodextrine (Cyclodextrin, Hydroxypropyl-Cyclodextrin, Sulfobutylester-Cyclodextrin) und/oder Phospholipide (z.B. hydrogeniertes Sojaphosphatidylcholin, Distearoylphosphatidylglycerol, L-Dimyristoylphosphatidylcholin, L-Dimyristoylphosphatidylglycerol)

Bei der Herstellung von intravenös zu verabreichenden Zubereitungen, d.h. Infusionslösungen, sind dies bevorzugt wässrige Lösungen, wobei es möglich ist, diese gebrauchsfertig vorzuhalten oder direkt vor Gebrauch herzustellen.

Erfindungsgemäß kann die pharmazeutische Zusammensetzung mit einer wässrigen zur intravenösen Gabe geeigneten üblichen Flüssigkeit, bevorzugt physiologischer Kochsalzlösung, einer 5-10%igen Glukose-Lösung, Ringer-Lösung, Ringer-Laktat-Lösung, Citrat-Puffer, gereinigtem Wassser und/oder HES zu einer Infusionslösung gemischt werden. Die entweder gebrauchsfertige Lösungen oder pharmazeutische Zusammensetzung (wenn die Infusionslösung erst direkt vor Gebrauch hergestellt wird) werden sterilisiert und sind anschließend bei Raumtemperaturen (< 25 °C) über Monaten haltbar. Die Sterilisierung erfolgt bevorzugt durch Sterilfiltration durch Filter sowie durch einen Autoklaven bei einer Temperatur von 120°C.

Nach einer Ausführungsform des erfindungsgemäßen Verfahrens wird eine entsprechende Menge 7β-Hydroxycholesterol in einem wasserlöslichen organischen Lösungsmittel (z.B. Propylenglykol und/oder DMSO und/oder Ethanol und/oder Aceton) durch leichtes Erwärmen bei 30-40 °C während 15 Minuten gelöst. Anschließend wird der Lösungsvermittler, d.h. PEG-40 hydriertes Rizinusöl oder polyethoxylierte 12-Hydroxystearinsäure, dazugegeben und das entstandene Konzentrat gut gemischt. Dieses Konzentrat wird zur gewünschten Menge, bevorzugt 100 ml, 200 ml und 400 ml, wässriger Flüssigkeit, bevorzugt Glucoselösung oder physiologischer Kochsalzlösung, gegeben. Diese Lösung wird steril filtriert und anschließend bei über 100°C, vorzugsweise 120 °C -140°C, sterilisiert.

Nach einer weiteren Ausführungsform des erfindungsgemäßen Verfahrens wird eine entsprechende Menge 7β-Hydroxycholesterol in einem wasserlöslichen organischen Lösungsmittel (z.B. Propylenglykol und/oder DMSO und/oder Aceton und/oder Ethanol) durch leichtes Erwärmen bei 30-40 °C während 15 Minuten gelöst. Anschließend wird der Lösungsvermittler, d.h. PEG-40 hydriertes Rizinusöl oder polyethoxylierte 12-Hydroxystearinsäure, dazugegeben und das entstandene Konzentrat gut gemischt. Dieses Konzentrat wird steril filtriert und anschließend bei über 100°C, vorzugsweise bei 120 °C - 140°C, sterilisiert. In einer bevorzugten Ausführungsform kann das Konzentrat mit einer wässrigen Lösung (z.B. Wasser, Citratpuffer, Kochsalzlösung) verdünnt werden. Durch die geringere Viskosität lässt sich die Lösung besser steril filtrieren. Die erfindungsgemäße Zusammensetzung wird zu gegebener Zeit, z.B. kurz vor der Gabe als Infusion, mit der entsprechenden Menge steriler wässriger Flüssigkeit, z.B. steriler physiologischer Kochsalzlösung oder Glukoselösung gemischt, bevorzugt 100 ml, 200 ml oder 400 ml.

Im Rahmen der Erfindung konnte die Stabilität von 7β-Hydroxycholesterol in Propylenglykol und/oder DMSO und/oder Ethanol sowie PEG 40 hydriertem Rizinusöl bzw. polyoxyethylierter 12-Hydroxystearinsäure (Kolliphor® HS15) nachgewiesen werden. Die Lösung von 7β-Hydroxycholesterol in Propylenglykol und/oder DMSO und/oder Ethanol sowie PEG 40 hydriertem Rizinusöl bzw. Kolliphor® HS15 war problemlos mit physiologischer Kochsalzlösung und/oder einer Glukoselösung mischbar. Es konnte auch nachgewiesen werden, dass 7β-Hydroxycholesterol in der so hergestellten Infusionslösung über Wochen und Monate stabil war.

Als besonders bevorzugt haben sich dabei die nachfolgenden Zusammensetzungen herausgestellt. Im Detail:
Propylenglykol
ggf. Ethanol 96%
PEG40 hydriertes Rizinusöl
7β-Hydroxycholesterol
Ethanol 96 %
Kolliphor® HS 15
Citratpuffer
7β-Hydroxycholesterol

Beide Zusammensetzungen können bevorzugt in physiologischer Kochsalzlösung aufgenommen werden.

Die Zugabe von Citratpuffer ist oftmals vorteilhaft, da hiermit ein gewünschter pH-Wert einfach einzustellen und beizubehalten ist.

Die erfindungsgemäße Zusammensetzung wird insbesondere als Infusion eingesetzt zur Behandlung von Tumoren aller Phänotypen, zur Durchbrechung von Resistenzen bei der Behandlung von Krebs, zur Steigerung der allgemeinen Immunabwehr, zur Behandlung bakterieller und viraler Erkrankungen, Autoimmunerkrankungen sowie erhöhten Stress- und Umweltbelastungen. Die Verabreichungen erfolgt gemäß Standardverfahren zur intravenösen Verabreichung, insbesondere als Infusion.

Als besonders vorteilhaft hat sich erwiesen, die Zusammensetzung als Infusion in einem zeitlichen Abstand, z.B. 2-6 Stunden, vorteilhafterweise etwa 4 Stunden, zur Gabe von Caroverinkapseln (1-[2-(diethylamino)ethyl]-3-(4-methoxybenzyl)quinoxalin- 2(1*H*)-on) oder Synoverin®-Kapseln (7β Hydroxycholesterol + Caroverin) zu verabreichen. Bevorzugt ist dabei die Gabe der erfindungsgemäßen Infusion ca. 4 Stunden vor der Caroverin- bzw. Synoverin®-Kapsel-Verabreichung.

### Beispiele

Die vorliegende Erfindung wird durch die nachfolgenden nicht einschränkenden Beispiele detailliert beschrieben.

### Beispiel 1:

| | |
|---|---|
| Ethanol 96 % | 0,3 g |
| Propylenglykol | 2,0 g |
| PEG40 hydriertes Rizinusöl | 0,5 g |
| 7β-Hydroxycholesterol | 0,01g |
| Physiologische Kochsalzlösung | ad 100 ml |

(oder 5%ige Glukoselösung)

### Beispiel 2:

| | |
|---|---|
| Propylenglykol | 20 g |
| PEG40 hydriertes Rizinusöl | 2 g |
| 7β-Hydroxycholesterol | 0,06 g |
| Physiologische Kochsalzlösung (oder 5%ige Glukoselösung) | ad 200 ml |

### Beispiel 3:

| | |
|---|---|
| Propylenglykol | 20 g |
| PEG40 hydriertes Rizinusöl | 2 g |
| 7β-Hydroxycholesterol | 0,04 g |
| Physiologische Kochsalzlösung (oder 5%ige Glukoselösung) | ad 400 ml |

### Beispiel 4:

| | |
|---|---|
| Propylenglykol | 7 g |
| PEG40 hydriertes Rizinusöl | 1,5 g |
| 7β-Hydroxycholesterol | 0,04 g |
| Physiologische Kochsalzlösung (oder 5%ige Glukoselösung) | ad 200 ml |

### Beispiel 5:

| | |
|---|---|
| Propylenglykol | 20 g |
| PEG40 hydriertes Rizinusöl | 1 g |
| 7β-Hydroxycholesterol | 0,04 g |
| Physiologische Kochsalzlösung | ad 50 ml |

### Beispiel 6:

| | |
|---|---|
| Dimethylsulfoxid (DMSO) | 20 g |
| PEG40 hydriertes Rizinusöl | 1 g |
| 7β-Hydroxycholesterol | 0,04 g |
| Physiologische Kochsalzlösung | ad 50 ml |

### Beispiel 7:

| | |
|---|---|
| Dimethylsulfoxid (DMSO) | 10 g |
| Propylenglykol | 10 g |
| PEG40 hydriertes Rizinusöl | 1 g |
| 7β-Hydroxycholesterol | 0,04 g |
| Physiologische Kochsalzlösung | ad 50 ml |

### Beispiel 8:

| | |
|---|---|
| Ethanol 96 % | 0,3 g |
| Kolliphor® HS 15 | 4,0 g |
| Citratpuffer | 4,0 g |
| 7β-Hydroxycholesterol | 0,04 g |
| Physiologische Kochsalzlösung (oder 5%ige Glukoselösung) | ad 100 ml |

### Beispiel 9:

| | |
|---|---|
| Ethanol 96 % | 0,3 g |
| Kolliphor® HS 15 | 4,0 g |
| Gereinigtes Wasser | 4,0 g |
| 7β-Hydroxycholesterol | 0,04 g |
| Physiologische Kochsalzlösung (oder 5%ige Glukoselösung) | ad 100 ml |

### Beispiel 10:

| | |
|---|---|
| Propylenglykol | 1,0 g |
| Kolliphor® HS 15 | 4,0 g |
| Gereinigtes Wasser | 4,0 g |
| 7β-Hydroxycholesterol | 0,04 g |
| Physiologische Kochsalzlösung (oder 5%ige Glukoselösung) | ad 100 ml |

### Beispiel 11:

| | |
|---|---|
| DMSO | 1,0 g |
| Kolliphor® HS 15 | 4,0 g |
| Gereinigtes Wasser oder Citratpuffer | 4,0 g |
| 7β-Hydroxycholesterol | 0,04 g |
| Physiologische Kochsalzlösung (oder 5%ige Glukoselösung) | ad 100 ml |

### Beispiel 12:

### Stabilitätsuntersuchungen:

Nach der Sterilisation bei 120 °C konnte weder beim Konzentrat noch bei der fertig zubereiteten Infusionslösung (physiologische Kochsalzlösung) eine Zersetzung des 7β-Hydroxycholesterols festgestellt werden. Das bedeutet, dass selbst bei dieser hohen thermischen Belastung das 7β-Hydroxycholesterol stabil blieb. Eine Lagerung des Konzentrats bei 40 °C während 3 Monaten zeigte ebenfalls keine Zersetzung des 7β-Hydroxycholesterols. Die Lösung wurde in Glasampullen gelagert.

Eine fertig zubereitete Infusionslösung (auf Basis von physiologischer Kochsalzlösung) wurde bei Raumtemperatur (< 25 °C) während 3 Monaten gelagert. Die Lösung blieb klar, es gab keine Ausflockung. Mittels HPLC konnte keine Abnahme des Gehalts an 7β-Hydroxycholesterol nach der Lagerung festgestellt werden.

## Patentansprüche

1. Pharmazeutische Zusammensetzung enthaltend (a) 7β-Hydroxycholesterol, (b) ein wasserlösliches organisches Lösungsmittel und (c) einen Lösungsvermittler, wobei der Lösungsvermittler PEG hydriertes Rizinusöl oder polyoxyethylierte 12-Hydroxystearinsäure ist.

2. Pharmazeutische Zusammensetzung nach Anspruch 1, wobei das wasserlösliche organische Lösungsmittel (b) Propylenglykol und/oder DSMO und/oder Ethanol und/oder Aceton ist.

3. Pharmazeutische Zusammensetzung nach Anspruch 1 oder 2, wobei sie folgende Zusammensetzung aufweist:
(a) 7β-Hydroxycholesterol 0,1-2,0 Gew.-%
(b) Wasserlösliches organisches Lösungsmittel 68-97 Gew.-%
(c) Lösungsvermittler 1-30 Gew.-%

4. Pharmazeutische Zusammensetzung nach einem der Ansprüche 1 bis 3, wobei sie folgende Zusammensetzung aufweist:
7β-Hydroxycholesterol 0,1-2,0 Gew.-%
Propylenglykol und/oder DMSO 68-97 Gew.-%
PEG hydriertes Rizinusöl 1-30 Gew.-%

5. Pharmazeutische Zusammensetzung nach einem der Ansprüche 1 bis 4, wobei sie weiter einen oder mehrere Alkohole und/oder Antioxidantien aufweist.

6. Pharmazeutische Zusammensetzung nach einem der Ansprüche 1 bis 5, wobei das PEG hydrierte Rizinusöl PEG40 hydriertes Rizinusöl ist.

7. Verfahren zur Herstellung einer pharmazeutischen Zusammensetzung nach einem der Ansprüche 1 bis 6, umfassend das Lösen von 7β-Hydroxycholesterol durch leichtes Erwärmen in dem wasserlöslichen organischen Lösungsmittel, vorzugsweise Propylenglykol und/oder DSMO, und Hinzugeben des Lösungsvermittlers, vorzugsweise PEG hydriertes Rizinusöl oder polyoxyethylierte 12-Hydroxystearinsäure.

8. Verfahren nach Anspruch 7, wobei die pharmazeutische Zusammensetzung steril filtriert und bei 120°C -140°C sterilisiert wird.

9. Verwendung der pharmazeutischen Zusammensetzung nach einem der Ansprüche 1-6 zur Herstellung einer intravenös zu verabreichenden Zubereitung.

10. Verwendung nach Anspruch 9, wobei die intravenös zu verabreichende Zubereitung eine Infusionslösung ist.

11. Verwendung nach Anspruch 9, wobei die Infusionslösung die pharmazeutische Zusammensetzung nach einem der Ansprüche 1-6 und eine wässrige Flüssigkeit enthält.

12. Verwendung nach Anspruch 11 wobei die wässrige Flüssigkeit physiologische Kochsalzlösung, Glucoselösung oder Ringer-Lösung ist.

## Claims

1. A pharmaceutical composition comprising (a) 7β-hydroxycholesterol, (b) a water soluble organic solvent, and (c) a solubilizing agent, wherein the solubilizing agent is PEG hydrated castor oil or polyoxyethylated 12-hydroxystearic acid.

2. The pharmaceutical composition according to claim 1, wherein the water soluble organic solvent (b) is propylene glycol and/or DMSO and/or ethanol and/or acetone.

3. The pharmaceutical composition according to claim 1 or 2, wherein the following composition comprises:
(a) 0.1-2.0% (w/w) 7β-hydroxycholesterol,
(b) 68-97% (w/w) water soluble organic solvent,
(c) 1-30% (w/w) solubilizing agent.

4. The pharmaceutical composition according to any of claims 1 to 3, wherein the following composition comprises:
0.1-2.0% (w/w) 7β-hydroxycholesterol,
68-97% (w/w) propylene glycol and/or DMSO,
1-30% (w/w) PEG hydrated castor oil.

5. The pharmaceutical composition according to any of claims 1 to 4, wherein the composition further comprises one or more alcohols and/or antioxidants.

6. The pharmaceutical composition according to any of claims 1 to 5, wherein the PEG hydrated castor oil is PEG40 hydrated castor oil.

7. A method for preparing a pharmaceutical composition according to any of claims 1 to 6, comprising dissolving 7β-hydroxycholesterol by moderate heating in the water soluble organic solvent, preferably propylene glycol and/or DMSO, and by adding the solubilizing agent, preferably PEG hydrated castor oil or polyoxyethylated 12-hydroxystearic acid.

8. The method according to claim 7, wherein the pharmaceutical composition is sterile filtered and is sterilized at 120°C-140°C.

9. Use of the pharmaceutical composition according to any of claims 1-6 for preparing an intravenously administered preparation.

10. The use of claim 9, wherein the intravenously administered preparation is an infusion solution.

11. The use of claim 9, wherein the infusion solution comprises the pharmaceutical composition according to any of claims 1-6 and an aqueous liquid.

12. The use of claim 11, wherein the aqueous liquid is physiological saline solution, glucose solution or Ringer's solution.

## Revendications

1. Composition pharmaceutique contenant (a) du 7β-hydroxycholestérol, (b) un solvant organique soluble dans l'eau et (c) un agent de solubilisation, dans laquelle le solubilisant est de l'huile de ricin hydrogénée PEG ou de l'acide 12-hydroxystéarique polyoxyéthylé.

2. Composition pharmaceutique selon la revendication 1, dans laquelle le solvant organique soluble dans l'eau (b) est le propylène-glycol et/ou le DSMO et/ou l'éthanol et/ou l'acétone.

3. Composition pharmaceutique selon la revendication 1 ou 2, dans laquelle elle présente la composition suivante:
(a) 7β-hydroxycholestérol de 0,1 à 2,0% en poids
(b) solvant organique soluble dans l'eau de 68 à 97% en poids
(c) solubilisant de 1 à 30% en poids

4. Composition pharmaceutique selon l'une des revendications 1 à 3, dans laquelle elle présente la composition suivante:
7β-hydroxycholestérol de 0,1 à 2,0% en poids
propylène-glycol et/ou DMSO de 68 à 97% en poids
huile de ricin hydrogénée PEG de 1 à 30% en poids

5. Composition pharmaceutique selon l'une des revendications 1 à 4, dans laquelle elle présente par ailleurs un ou plusieurs alcools et/ou antioxydants.

6. Composition pharmaceutique selon l'une des revendications 1 à 5, dans laquelle l'huile de ricin hydrogénée PEG est une huile de ricin hydrogénée PEG40.

7. Procédé de préparation d'une composition pharmaceutique selon l'une des revendications 1 à 6, comprenant le fait de dissoudre le 7β-hydroxycholestérol en le chauffant légèrement dans le solvant organique soluble dans l'eau, de préférence le propylène-glycol et/ou le DMSO, et en ajoutant l'agent de solubilisation, de préférence l'huile de ricin hydrogénée PEG ou l'acide 12-hydroxystéarique polyoxyéthylé.

8. Procédé selon la revendication 7, dans lequel la composition pharmaceutique est filtrée de manière stérile et stérilisée à de 120°C à 140°C.

9. Utilisation de la composition pharmaceutique selon l'une des revendications 1 à 6 pour la préparation d'une préparation à administrer par voie intraveineuse.

10. Utilisation selon la revendication 9, dans laquelle la préparation à administrer par voie intraveineuse est une solution d'infusion.

11. Utilisation selon la revendication 9, dans laquelle la solution d'infusion contient la composition pharmaceutique selon l'une des revendications 1 à 6 et un liquide aqueux.

12. Utilisation selon la revendication 11, dans laquelle le liquide aqueux est une solution saline physiologique, une solution de glucose ou une solution de Ringer.
